# EUROPÄISCHE PATENTSCHRIFT

(11) **EP 3 077 495 B1**
(45) Veröffentlichungstag und Bekanntmachung des Hinweises auf die Patenterteilung: **11.11.2020**
(21) Anmeldenummer: 14806616.0
(22) Anmeldetag: 02.12.2014
(51) Int. Cl.: C12M 1/00, C12M 1/24, C12M 3/00, C12M 1/12

(54) **AUFRÜSTSET FÜR BIOREAKTOREN ZUR DURCHFÜHRUNG DER MIKROBIELLEN BIOELEKTROSYNTHESE**
EXPANSION KIT FOR BIOREACTORS USED FOR PERFORMING MICROBIAL BIO-ELECTROSYNTHESIS
KIT POUR BIORÉACTEURS POUR LA RÉALISATION DE LA BIOÉLECTRO-SYNTHÈSE MICROBIENNE

(30) Priorität: 02.12.2013 DE 102013224673
(43) Veröffentlichungstag der Anmeldung: 12.10.2016
(73) Patentinhaber: Helmholtz-Zentrum für Umweltforschung GmbH - UFZ, 04318 Leipzig (DE)
(72) Erfinder: HARNISCH, Falk, 04275 Leipzig (DE); HUNGER, Steffi, 04827 Machern (DE); ZEHNSDORF, Andreas, 04347 Leipzig (DE); BEYER, Daniel, 04509 Delitzsch (DE); FILIPE MORGADO ROSA, Luis, 04347 Leipzig (DE)
(74) Vertreter: Gulde & Partner
(86) Internationale Anmeldenummer: PCT/EP2014/076293
(87) Internationale Veröffentlichungsnummer: WO 2015/082490

(56) Entgegenhaltungen:
- EP-A1- 2 653 531
- EP-A2- 0 170 098
- EP-A2- 2 151 491
- WO-A1-00/03447
- WO-A1-2007/037407
- WO-A1-2010/117864
- WO-A1-2013/120853
- WO-A2-2005/003284
- WO-A2-2009/087448
- WO-A2-2011/062485
- DE-A1- 2 018 289
- DE-A1- 10 350 972
- DE-A1-102011 054 364
- US-A- 4 296 205
- US-A- 4 772 558
- US-A1- 2013 256 149

## Beschreibung

Die vorliegende Erfindung betrifft ein Aufrüstset für Bioreaktoren zur Durchführung einer mikrobiellen Bioelektrosynthese, einen Bioreaktor und dessen Verwendung.

Die mikrobielle Bioelektrosynthese oder mikrobielle elektrochemische Technologie erfährt derzeit weltweit einen erheblichen Aufschwung. Ziel der mikrobiellen Bioelektrosynthese ist die Verknüpfung der mikrobiellen Syntheseleistung vorzugsweise über extrazellulären Elektronentransfer mit dem Fluss von elektrischem Strom. Dabei kann sowohl elektrischer Strom bei Oxidationsprozessen an Anoden als auch bei Reduktionen an der Kathode umgesetzt werden. In beiden Fällen ist die mikrobielle Stoffumwandlung mit dem Fluss elektrischen Stroms verbunden.

Dabei hat die mikrobielle Bioelektrosynthese im Vergleich zur klassischen elektrochemischen Synthese zahlreiche Vorteile wie potentiell höhere Selektivitäten, Ausbeuten etc., aber auch gegenüber enzymatischen Bioelektrosynthesen bestehen Vorteile, da hier zumeist nur max. 2 Elektronen pro Reaktionsschritt übertragen werden können und der Katalysator nicht selbstregenerierend ist.

Der Begriff der mikrobiellen Bioelektrosynthese kann hier drei wesentliche Klassen von Prozessen einschließen, dazu zählen:
i) die Synthese aus CO₂,
ii) die "Veredlung" von Vorstufen, und
iii) die elektrochemische Fermentationssteuerung.

Das Produktspektrum ist dabei potentiell sehr vielfältig.

Forschungen auf diesem Gebiet, insbesondere in verfahrenstechnischer Hinsicht, befinden sich bezüglich der verwendeten Technik im Anfangsstadium und werden vorwiegend in Laborversuchen von geringer Größe von beispielsweise 100 mL sowie in "Selbstbaureaktoren" mit einer Größe von zumeist < 1 L durchgeführt, wobei keine oder nur eine geringe Prozessüberwachung und -steuerung realisiert wird.

Da jedoch gleichzeitig das weltweite Interesse an der mikrobiellen Biöelektrosynthese steigt, ist von einem steigenden Bedarf an geeigneter Bioreaktortechnik auszugehen, die vergleichbare Ergebnisse zulässt. Aus diesem Grund wird auch der Bedarf an vergleichbaren Laborreaktorsystemen zur Durchführung von Versuchen der mikrobiellen Elektrosynthese steigen.

Die Bezeichnung Membranreaktor ist im technischen Sprachgebrauch sehr weit gefasst. So werden darunter zum Beispiel Reaktoren mit Keramikmembranrohren, um verschiedene Stoffe chemisch miteinander reagieren zu lassen, ebenso verstanden wie die Vielzahl von Reaktoren mit Membranen zur Rückhaltung von Mikroorganismen in der Abwasserbehandlung. Diese sind nicht zur Durchführung der mikrobiellen Bioelektrosynthese geeignet WO2013120853 offenbart ein Kulturgefäß für Mikroorganismen, welches ausgerüstet ist mit mindestens einem Trennelement, das das Kulturgefäß in mindestens zwei separate Bioreaktorkammern, die nach oben eine Öffnung aufweisen, unterteilt. Im Trennelement ist eine Element mit Filter- und Siebfunktion angeordnet, der gelöste Partikel in den unterteilten Bereichen zurückhält DE102011054364 offenbart einen Bioreaktor zur Zellkultivierung. Im Deckel, mit dem der Reaktorbehälter verschlossen ist, sind mehrere Durchführungen gebildet, um die für den Prozess der Kultivierung notwendigen Stoffe bereitzustellen und/oder Stoffe aus dem Reaktorbehälter abzuführen, zum Beispiel beim Kultivieren auftretende Gas. Die Druckschriften DE2018289 und EP0170098 offenbaren jeweils einen becher- oder kegelförmigen Einsatz zum Einsetzen in einen Bioreaktor, wobei der Einsatzbehälter auf einer Seite offen ist und ein Fenster besitzt, dass mit einer Membrane versehen ist.

Reaktoren, die zur Durchführung der mikrobiellen Bioelektrosynthese geeignet sind, werden beispielsweise in den Druckschriften US 2013/0256149 A1, WO 2011/062485 A2, WO 2010/117864 A1 und WO 00/03447 offenbart, wobei es sich hierbei um "stand-alone" Systeme handelt, die nicht für eine Integration in bestehende/ vorhandene Bioreaktorsysteme geeignet sind, so dass diese nicht direkt mit der in bestehenden Infrastrukturen vorhandenen Analytik verbunden werden und die dort vorhandene Expertise oder Skalierung nutzen können.

Es ist daher Aufgabe der Erfindung, ein Aufrüstset für Bioreaktoren zu schaffen, das eine Erweiterung von Bioreaktoren nach dem Stand der Technik als Elektrobioreaktoren erlaubt, so dass deren Nutzung für die mikrobielle Bioelektrosynthese ermöglicht wird. Diese Aufgabe wird durch ein Aufrüstset für Bioreaktoren mit den Merkmalen des Anspruchs 1 gelöst.

Dazu ist es erfindungsgemäß vorgesehen, ein Aufrüstset gemäß Anspruch 1 für Bioreaktoren zur Durchführung einer mikrobiellen Bioelektrosynthese zu schaffen, das eine an einer Seite, vorzugsweise der Oberseite, offene Reaktionskammer aufweist, die in einer Seitenwand ein vorzugsweise rechtwinkliges Fenster bzw. eine entsprechende Durchgangsöffnung besitzt, das mit einer vorzugsweise reversibel festgelegten Membran versehen ist.

Bei der Membran handelt es sich um eine ionenselektive Membran, beispielsweise eine Nafion-Membran oder eine keramische Membran. Geeignete Membranen sind dem Fachmann grundsätzlich bekannt.

Nach einer bevorzugten Ausführungsform weist die Reaktionskammer einen kreissegmentförmigen Querschnitt auf, wobei im Bereich der Kreissehne das Fenster ausgebildet ist.

Die Seitenfläche, die sich durch die Kreissehne ergibt, kann auch anders beschaffen sein, so kann sich in der Wandung der Reaktionskammer auch eine gerundete oder eckige Einbuchtung mit einem Fenster an der tiefsten Stelle befinden. Entscheidend ist, dass durch die Ausgestaltung der Reaktionskammer eine zweite Reaktionskammer im nachzurüstenden Bioreaktor geschaffen wird, der üblicherweise einen runden Querschnitt besitzen. Bevorzugt ist dabei die Ausbildung einer planen Seitenwand zur Aufnahme des Fensters bzw. der Durchgangsöffnung.

Reaktoren sind üblicherweise im Wesentlichen zylindrisch ausgestaltet, so dass das Aufrüstset bzw. die Reaktionskammer dicht an der Wandung des Reaktors anliegt. Daher ist eine Temperierung der beiden Reaktionskammern problemlos möglich. Ansonsten sitzt die eingebrachte Reaktionskammer des Aufrüstsets auf dem Boden des nachgerüsteten Bioreaktors auf.

Das Volumen der beiden Reaktionskammern steht besonders bevorzugt im Verhältnis 1:1, aber auch Verhältnisse bis 1: 4 werden vorzugsweise eingesetzt, um eine mikrobielle Bioelektrosynthese durchzuführen.

Zur reversiblen Festlegung der Membran ist diese vorzugsweise zwischen einer Dichtung und einem Rahmen angeordnet, wobei der Rahmen die Membran und die Dichtung an der Reaktionskammer derart fixiert, dass das Fenster überdeckt ist.

In welcher Weise die Membran mittels des Rahmens fixiert wird, ist dem Fachmann grundsätzlich bekannt. So kann dieser beispielsweise verschraubt oder geklebt sein oder auch mittels einer klemmenden Verbindung gehalten werden.

Die Größe des Fensters und damit der Membran wird derart gewählt, dass der Innenwiderstand möglichst gering ist. Zweckmäßigerweise liegt daher das Verhältnis der Größe der Elektroden, die jeweils in den beiden Reaktionskammern des Bioreaktors angeordnet sind, zur Größe des Fensters bzw. der Membran im Bereich von 1:100 bis 100:1.

Das erfindungsgemäße Aufrüstset wird in eine Reaktionskammer eines üblichen, Bioreaktors nach dem Stand der Technik eingebracht und unterteilt damit diesen Bioreaktor in zwei durch die Membran getrennte Reaktionskammern, so dass der nachgerüstete Bioreaktor bei einer mikrobiellen Elektrosynthese, beispielsweise einer kathodischen Hydrierung, vorzugsweise unter Nutzung von mikrobiellem Extrazellulären Elektronentransfers, verwendbar ist.

Erfindungsgemäß ist bei dem Aufrüstset auch ein Reaktordeckel vorgesehen, der die Reaktorkammer des Ausrüstsets sowie den ursprünglichen Bioreaktor verschließt, wobei vorzugsweise eine Dichtung zwischen der Reaktorkammer des Nachrüstsets und dem Deckel vorgesehen ist.

Der Reaktordeckel weist zudem zumindest zwei Durchgangsöffnungen für Rührer, Elektroden, Messsonden, Begasung oder Entgasung und dergleichen auf.

Der Reaktordeckel weist übliche Befestigungsmittel auf, um beispielsweise ein Verschrauben mit dem nachgerüsteten Bioreaktor zu ermöglichen.

Die Reaktionskammer besteht vorzugsweise aus einem elektrochemisch inerten Material. Dies ist vorzugsweise Glas, Keramik oder ein sterilisierbarer Kunststoff, wie PTFE oder PEEK.

Vorteilhafterweise ist das Aufrüstset für Bioreaktoren zahlreicher Hersteller geeignet, da dieses vorzugsweise auf eine Größe von nachzurüstenden Bioreaktoren von 2L ausgelegt ist, die weit verbreitet ist. Das erfindungsgemäße Aufrüstset ist jedoch auch für andere Reaktorgrößen geeignet, wie beispielsweise im Bereich von 0,3 bis 5L und kann entsprechend ausgelegt werden.

Mit Hilfe dieses erfindungsgemäßen Aufrüstsets lassen sich Bioreaktoren verschiedener Hersteller so erweitern, dass in diesen vorteilhafterweise vergleichbare Untersuchungen zur mikrobiellen Bioelektrosynthese im Labormaßstab durchgeführt werden können. Damit können Forschungs- und Entwicklungsarbeiten zur mikrobiellen Bioelektrosynthese systematisch unter kontrollierten und vergleichbaren Bedingungen durchgeführt werden.

Von Vorteil ist zudem, dass auf bereits vorhandene Biorektoren, dazugehörige Regelungstechnik und Peripherie zurückgegriffen werden kann und Standardbioreaktoren bei Bedarf in Bioreaktoren für die Bioelektrosynthese umgerüstet werden können.

Besonders vorteilhaft ist, dass diese Umrüstung reversibel ist, wodurch sich teure Doppelanschaffungen vermeiden lassen.

Besonders vorteilhaft ist zudem, dass diese Integration in existierende Bioreaktorensysteme auch dem Nicht-Fachmann, insbesondere dem Nicht-Elektrochemiker eine Durchführung solcher Arbeiten ermöglicht wird.

Weiterhin ist es sehr vorteilhaft - und durch bisherige Bioreaktorsysteme nach dem Stand der Technik in keiner Weise gegeben -, dass eine weitgehend problemlose Anschlussfähigkeit des erfindungsgemäß nachgerüsteten Reaktors im 2L-Maßstab an den industriellen Maßstab, beispielsweise im 400L Maßstab, sowie dessen Skalierbarkeit gegeben ist.

Vorteilhafterweise lassen sich mittels des erfindungsgemäßen Nachrüstsets alle Arten von mikrobiellen Bioelektrosynthesen durchführen. Dazu zählen alle Redoxreaktionen, d.h. Biotransformationen mit Veränderung des Redoxstatus des Zielmoleküls, sowohl oxidative (anodische) als auch reduktive (kathodische) Prozesse. Diese schließen, beispielsweise folgende Reaktionen, welche einen besonderen Mehrwert darstellen können mit ein:
1. Mehrstufige (bio)elektrochemische Oxidationen und Reduktionen sowie die Knüpfung von C-C-Bindungen (welche nicht von konventionellen chemischen und enzymatischen Katalysatoren realisiert werden können),
2. Selektive Oxidationen (Vermeidung von Überoxidation wie beim Einsatz anderer Oxidationsmittel wie Luftsauerstoff),
3. Hydrierungen beim "milden" Reaktionsbedingungen (Temperaturen von max. 100°C, keine H₂-Atmosphären),
4. elektrochemische Fermentationen (Erhöhung der Ausbeute durch substöchiometrischen Einsatz elektrischer Energie),
5. Reduktion/ Fixierung von CO₂ in C₁ und C₁₊ₙ-Körpern.
6. Einsatz von "elektroaktiven" Mikroorganismen als Katalysatoren mit einer vorzugsweisen Chemo-, Regio-, und Stereospezifität.

Vorteilhafterweise lassen sich in mit dem Aufrüstset versehenen Bioreaktoren zudem sowohl planktonische Prozesse als auch Biofilmprozesse durchführen.

Gegenstand der Erfindung ist zudem ein Bioreaktor zur Durchführung der mikrobiellen Bioelektrosynthese ein Aufrüstset aufweisend. Dieser Bioreaktor weist die bei den nachzurüstenden Bioreaktoren beschriebenen Merkmale auf.

Unter einem Bioreaktor im Rahmen der Erfindung wird ein Bioreaktor im technischen Maßstab verstanden, der sich mindestens durch eine minimale technische Ausstattung auszeichnet und die Durchführung einer mikrobiellen Bioelektrosynthese ermöglicht.

Die minimale technische Ausstattung umfasst vorzugsweise eine Prozesssteuerung vorzugsweise der Parameter Temperierung, Rührerdrehzahl, Art und Menge der Begasung sowie pH und den Einbau von Anode und Kathode zur Steuerung der Bioelektrosynthese über einen Potentiostaten bzw. eine Gleichspannungs- oder Gleichstromquelle.

So kann eine gute Durchmischung in den beiden durch dass Aufrüstset gegebenen Kammern stattfinden, um die sonst zwangsläufig auftretenden Gradienten im Reaktionsraum (Temperatur-, Konzentrationsgradienten etc.) zu vermeiden. Diese würden unweigerlich zu Produktivitätsverlusten führen.

Die beiden durch eine Membran getrennten Kammern erfüllen, bei Verwendung des Aufrüstsets, beide die Kriterien eines Bioreaktors im technischen Maßstab. Dies gilt dementsprechend auch für das Aufrüstset allein.

Zudem muss vorzugsweise eine Sterilisierbarkeit bei mindestens 121°C gegeben sein.

Vorteilhafte Weiterbildungen der Erfindung sind in den Unteransprüchen angegeben und in der Beschreibung beschrieben.

### Zeichnungen

Nachstehend wird die Erfindung anhand von Zeichnungen und der nachfolgenden Beschreibung näher erläutert. Es zeigen
- Fig. 1: in einer schematischen Darstellung eine mikrobielle Elektrosynthese am Beispiel einer kathodischen Hydrierung unter Nutzung von mikrobiellem extrazellulären Elektronentransfers nach dem Stand der Technik, und
- Fig. 2: in einer Explosivdarstellung ein Aufrüstset für Bioreaktoren zur Durchführung der Bioelektrosynthese.

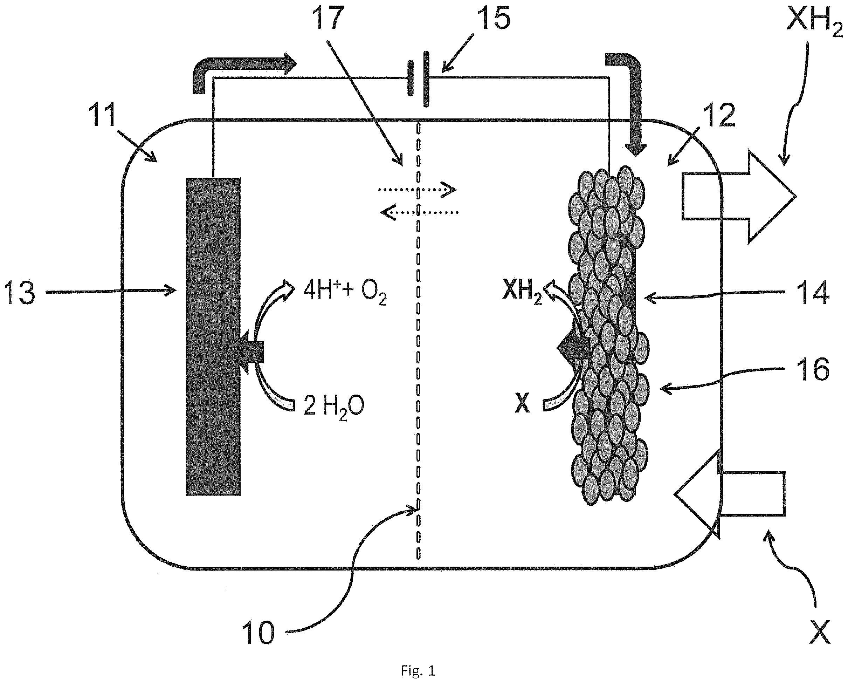

Mittels Fig. 1 wird beispielhaft eine mikrobielle Elektrosynthese nach dem Stand der Technik dargestellt. Es handelt sich um eine kathodische Hydrierung unter Nutzung eines mikrobiellen extrazellulären Elektronentransfers. In zwei voneinander durch eine Membran 10 getrennten Reaktionskammern 11, 12 ist jeweils eine Anode 13 bzw. eine Kathode 14 angeordnet, die mit einer Spannungsquelle 15 verbunden sind, wobei die Kathode 14 mit einem Biofilm 16 eines bioelektrokatalytisch aktiven Mikroorganismus versehen ist. An der Kathode 14 wird ein Substrat X selektiv zum Produkt XH₂ reduziert, während an der Anode 13 H₂O zur Bereitstellung von H⁺ und Elektronen oxidiert wird. Durch die Membran 10 erfolgt der entsprechende lonentransfer 17. Das erhaltene Produkt XH₂ wird aus der Reaktionskammer 12 abgeführt, während Substrat X zugeführt wird.

Für biotechnologisch interessante Konzepte ist vor allem die Chemo-, Regio-, und Stereospezifität der Bioelektrosynthese von großem Wert. Dabei wird das Reaktionssubstrat bzw. der Biofilm nicht als Wachstumssubstrat für den Biofilm verwendet, sondern ein separates Wachstumssubstrat sorgt für stabile stationäre Eigenschaften des Biofilms. Analog können auch anodische Konzepte, d.h. zur mikrobiellen Elektrooxidation, entwickelt werden.

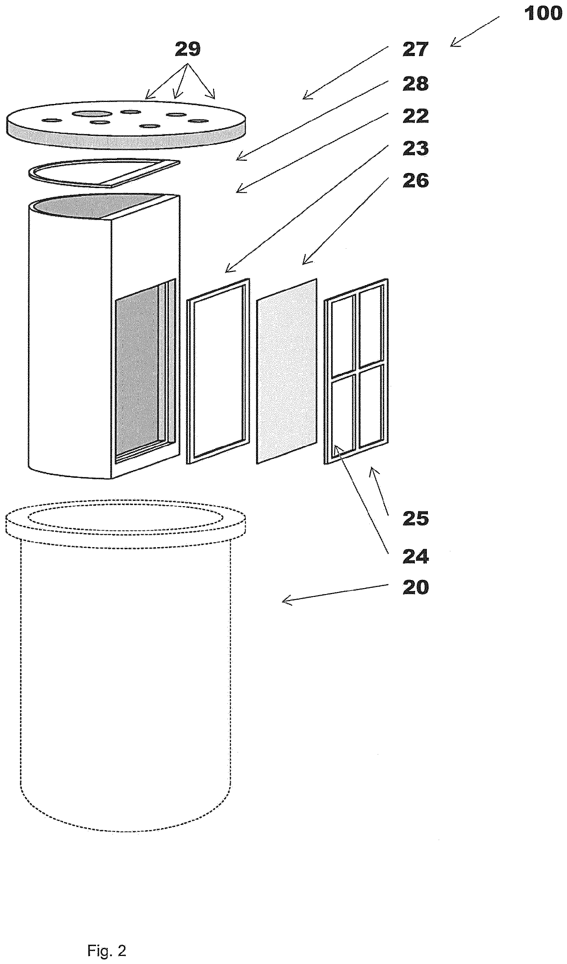

In Fig. 2 ist ein erfindungsgemäßes Aufrüstset 100 für Bioreaktoren 20 für die Durchführung der mikrobiellen Bioelektrosynthese dargestellt. Das Aufrüstset 100 weist eine Reaktorkammer 21 auf, die einen Kreissegmentförmigen Querschnitt besitzt. An der dadurch gegebenen flachen Seitenwand 22 der Reaktorkammer 21 ist ein rechteckiges Fenster 23 angeordnet, das mit einer Membran 24 verschließbar ist. Dazu wird die Membran 24 mit einem Rahmen 25 an der Reaktorkammer 21 fixiert, wobei zudem zwischen der Membran 24 und der Reaktorkammer 21 eine Dichtung 26 angeordnet ist. Die Reaktionskammer 21 wird in einen aufzurüstenden Reaktor 20 eingebracht und anschließend wird der Reaktor 20 mittels eines zum Aufrüstset 100 gehörenden Deckels 27 verschlossen, wobei zwischen der Reaktorkammer 20 und dem Deckel 27 eine Dichtung 28 angeordnet wird. Im Deckel 27 sind verschiedene Durchgangsöffnungen 29 vorgesehen, die für Elektroden, Rührer, Messsonden, Begasung, Entgasung und dergleichen bestimmt sind.

**Bezugszeichenliste**

| | |
|---|---|
| Membran | 10 |
| Reaktionskammer | 11, 12 |
| Anode | 13 |
| Kathode | 14 |
| Spannungsquelle | 15 |
| Biofilm | 16 |
| Ionentransfer | 17 |
| | |
| Substrat | X |
| Produkt | XH₂ |
| | |
| Aufrüstset | 100 |
| Bioreaktor | 20 |
| Reaktorkammer | 21 |
| flache Seitenwand | 22 |
| rechteckiges Fenster | 23 |
| Membran | 24 |
| Rahmen | 25 |
| Dichtung | 26 |
| Deckel | 27 |
| Dichtung | 28 |
| Durchgangsöffnung | 29 |

## Patentansprüche

1. Aufrüstset zum reversiblen Einbringen in einen Bioreaktor zur Unterteilung eines Bioreaktors in zwei getrennte Reaktionskammern und zur Durchführung einer mikrobiellen Bioelektrosynthese, wobei das Aufrüstset (100) eine an der Oberseite offene Reaktionskammer (21) aufweist, die in einer Seitenwand (22) ein Fenster (23) besitzt, das mit einer Membran (24) versehen ist, wobei das Aufrüstset ausgebildet ist, dass mittels der Membran (24) die Reaktionskammer (21) des Aufrüstsets im in den Bioreaktor eingebrachten Zustand mit einer Reaktionskammer des Bioreaktors verbunden ist, wobei das Aufrüstset (100) einen Reaktordeckel (27) aufweist, der zumindest zwei Durchgangsöffnungen (29) aufweist, in die Elektroden derart einbringbar sind, dass diese jeweils in eine der durch die Membran getrennten Reaktionskammern ragen und jeweils als Anode oder Kathode fungieren

2. Aufrüstset nach Anspruch 1, **dadurch gekennzeichnet, dass** die Membran (24) ionenselektiv ist.

3. Aufrüstset nach Anspruch 1 oder 2, **dadurch gekennzeichnet, dass** die Membran (24) mittels eines Rahmens (25) an der Reaktionskammer (21) fixiert ist.

4. Aufrüstset nach einem der Ansprüche 1 bis 3, **dadurch gekennzeichnet, dass** die Reaktionskammer (21) einen kreissegmentförmigen Querschnitt aufweist, wobei im Bereich der Kreissehne des Kreissegments das Fenster (23) ausgebildet ist.

5. Aufrüstset nach einem der Ansprüche 1 bis 3, **dadurch gekennzeichnet, dass** die Reaktionskammer (21) eine gerundete oder eckige Einbuchtung aufweist, die mit einem Fenster (23), vorzugsweise an der tiefsten Stelle der Einbuchtung versehen ist.

6. Aufrüstset nach einem der Ansprüche 1 bis 5, **dadurch gekennzeichnet, dass** die Reaktionskammer (21) aus einem elektrochemisch inerten Material besteht.

7. Bioreaktor ein Aufrüstset (100) nach einem der Ansprüche 1 bis 6 aufweisend.

8. Bioreaktor nach Anspruch 7, **dadurch gekennzeichnet, dass** der Bioreaktor (20) durch das Aufrüstset (100) zwei Reaktionskammern (20; 21) aufweist, wobei die zwei Reaktionskammern (20; 21) jeweils die Kriterien eines separaten Bioreaktors im technischen Maßstab aufweisen.

9. Bioreaktor nach Anspruch 8, **dadurch gekennzeichnet, dass** in beiden Reaktionskammern (21) mindestens eine Elektrode angeordnet ist.

10. Bioreaktor nach Anspruch 9, **dadurch gekennzeichnet, dass** das Verhältnis der Größe der Elektroden zur Größe der Membran (24) im Bereich von 1:100 bis 100:1 liegt.

11. Verwendung eines Bioreaktors nach einem der Ansprüche 7 bis 10 zur Durchführung von mikrobiellen Bioelektrosynthesen.

12. Verwendung eines Bioreaktors nach einem der Ansprüche 7 bis 10 zur Durchführung von bioelektrochemischen planktonischen Prozessen oder bioelektrochemischen Biofilmprozessen.

## Claims

1. An expansion kit for reversible incorporation in a bioreactor for dividing a bioreactor into two separate reaction chambers and for performing a microbial bio-electrosynthesis, wherein the expansion kit (100) has a reaction chamber (21) which is open on the top side and which has a window (23) in a sidewall (22), said window being provided with a membrane (24), wherein the expansion kit is formed in such a way that the reaction chamber (21) of the expansion kit is connected to a reaction chamber of the bioreactor by means of the membrane (24) when in a state of being incorporated in the bioreactor, wherein the expansion kit (100) has a reactor cover (27) which has at least two through openings (29) into which electrodes are insertable such that these electrodes each protrude into one of the reaction chambers which are separated by the membrane and function, respectively, as anode or cathode.

2. The expansion kit according to Claim 1, **characterized in that** the membrane (24) is ion-selective.

3. The expansion kit according to Claim 1 or 2, **characterized in that** the membrane (24) is fixed to the reaction chamber (21) by means of a frame (25).

4. The expansion kit according to any one of Claims 1 to 3, **characterized in that** the reaction chamber (21) has a circular segment-shaped cross section, wherein the window (23) is formed in the area of the chord of the circular segment.

5. The expansion kit according to any one of Claims 1 to 3, **characterized in that** the reaction chamber (21) has a rounded or angular indentation which is provided with a window (23), preferably at the lowest point of the indentation.

6. The expansion kit according to any one of Claims 1 to 5, **characterized in that** the reaction chamber (21) consists of an electrochemically inert material.

7. A bioreactor having an expansion kit (100) according to any one of Claims 1 to 6.

8. The bioreactor according to Claim 7, **characterized in that** the bioreactor (20) has two reaction chambers (20; 21) due to the expansion kit (100), wherein the two reaction chambers (20; 21) respectively meet the criteria of a separate bioreactor on a technical scale.

9. The bioreactor according to Claim 8, **characterized in that** at least one electrode is arranged in both reaction chambers (21).

10. The bioreactor according to Claim 9, **characterized in that** the ratio of the size of the electrodes to the size of the membrane (24) is in the range of from 1:100 to

11. A use of a bioreactor according to any one of Claims 7 to 10 for performing microbial bio-electrosyntheses.

12. The use of a bioreactor according to any one of Claims 7 to 10 for carrying out bio-electrochemical planktonic processes or bio-electrochemical biofilm processes.

## Revendications

1. Kit destiné à être placé de manière réversible dans un bioréacteur afin de diviser un bioréacteur en deux chambres de réaction séparées et de réaliser une bio-électrosynthèse microbienne, le kit (100) présentant une chambre de réaction (21) ouverte au niveau de la face supérieure et qui comporte, dans une paroi latérale, (22) une fenêtre (23) qui est munie d'une membrane (24), le kit étant configuré de telle sorte que, à l'aide de la membrane (24), la chambre de réaction (21) du kit dans l'état introduit dans le bioréacteur est reliée à une chambre de réaction du bioréacteur, le kit (100) présentant un couvercle de réacteur (27) qui présente au moins deux trous débouchants (29) dans lesquels des électrodes peuvent être introduites de telle sorte que ces dernières font saillie respectivement dans une des chambres de réaction séparées par la membrane et servent respectivement d'anode ou de cathode.

2. Kit selon la revendication 1, **caractérisé en ce que** la membrane (24) est ion-sélective.

3. Kit selon la revendication 1 ou 2, **caractérisé en ce que** la membrane (24) est fixée à la chambre de réaction (21) à l'aide d'un cadre (25).

4. Kit selon l'une quelconque des revendications 1 à 3, **caractérisé en ce que** la chambre de réaction (21) présente une section transversale en forme de segment circulaire, la fenêtre (23) étant configurée au niveau de la corde du segment circulaire.

5. Kit selon l'une quelconque des revendications 1 à 3, **caractérisé en ce que** la chambre de réaction (21) présente un évidement arrondi ou angulaire qui est muni d'une fenêtre (23), de préférence à l'endroit le plus profond de l'évidement.

6. Kit selon l'une quelconque des revendications 1 à 5, **caractérisé en ce que** la chambre de réaction (21) est composée d'un matériau électrochimiquement inerte.

7. Bioréacteur présentant un kit (100) selon l'une quelconque des revendications 1 à 6.

8. Bioréacteur selon la revendication 7, **caractérisé en ce que** le bioréacteur (20) présente deux chambres de réaction (20 ; 21) à travers le kit (100), les deux chambres de réaction (20 ; 21) présentant respectivement les critères d'un bioréacteur séparé à l'échelle technique.

9. Bioréacteur selon la revendication 8,
**caractérisé en ce qu'**au moins une électrode est disposée dans les deux chambres de réaction (21).

10. Bioréacteur selon la revendication 9, **caractérisé en ce que** le rapport de la grandeur des électrodes sur la grandeur de la membrane (24) est compris dans la plage de 1:100 à

11. Utilisation d'un bioréacteur selon l'une quelconque des revendications 7 à 10 pour la réalisation de bio-électrosynthèses microbiennes.

12. Utilisation d'un bioréacteur selon l'une quelconque des revendications 7 à 10 pour la réalisation de processus planctoniques bio-électrochimiques ou de processus de biofilms bio-électrochimiques.
